# EUROPÄISCHE PATENTSCHRIFT

(11) **EP 1 945 101 B1**
(45) Veröffentlichungstag und Bekanntmachung des Hinweises auf die Patenterteilung: **17.08.2011**
(21) Anmeldenummer: 06818471.2
(22) Anmeldetag: 10.11.2006
(51) Int. Cl.: A61B 5/15

(54) **STECHELEMENT, STECHSYSTEM UND VERFAHREN ZUR HAUTDETEKTION**
PRICKING ELEMENT, PRICKING SYSTEM, AND SKIN DETECTION METHOD
ELEMENT DE PONCTION, SYSTEME DE PONCTION ET PROCEDE DE DETECTION DE LA PEAU

(30) Priorität: 10.11.2005 EP 05024477
(43) Veröffentlichungstag der Anmeldung: 23.07.2008
(73) Patentinhaber: F. Hoffmann-La Roche AG, 4070 Basel (CH); Roche Diagnostics GmbH, 68305 Mannheim (DE)
(72) Erfinder: CALASSO, Irio Giuseppe, CH-6415 Arth (CH); KORNER, Stephan, CH-6330 Cham (CH); BRÜNDLER, Oliver, 6043 Adligenswil (DE); WAHL, Hans-Peter, 79650 Schopfheim (DE); GLAUSER, Michael, CH-6343 Rotkreuz (CH)
(74) Vertreter: Pfiz, Thomas
(86) Internationale Anmeldenummer: PCT/EP2006/010802
(87) Internationale Veröffentlichungsnummer: WO 2007/054335

(56) Entgegenhaltungen:
- WO-A-93/21974
- WO-A-2005/096941
- US-A1- 2005 197 666
- US-B1- 6 537 242

## Beschreibung

Die Erfindung betrifft ein Stechelement zur Gewinnung von Körperflüssigkeit durch bzw. über die Haut, insbesondere als Einwegteil für Blutzuckertests, mit einem Träger und einem starr daran abstehenden, in einer Stechbewegung in die Haut einstechbaren Stechorgan. Die Erfindung betrifft weiter ein Stechsystem zum Einsatz eines solchen vorzugsweise disposiblen Stechelements und ein Verfahren zur Hautdetektion für eine solche Probennahme.

Aus der WO 2005/096941 A1 ist ein System zur Blutentnahme mittels eines eine trägergebundene Sammeleinheit aufweisenden Stechelements bekannt, bei dem durch einen geräteseitig gelagerten Druckring auf einen dagegen gedrückten Finger ein Druck ausgeübt wird, um in der komprimierten Fingerpartie ausreichend Blut bereit zu stellen und anschließend durch Druckreduzierung das Austreten von Blut zu vermeiden. Zur Vermeidung einer Kontamination mit Blut und für die ausreichende Erhöhung des Körperinnendrucks muss der Druckring einen Durchmesser in der Größe der Fingerbeere besitzen, wodurch das Körperteil im Einstechbereich ausgewölbt wird. Durch die beim Stechvorschub auftreffende Nadel erfolgt dann eine Hauteindellung nach innen, bis schließlich abhängig vom Hauttyp und der Hautdicke des Probanden ein Stichkanal erzeugt wird.

Ausgehend hiervon liegt der Erfindung die Aufgabe zugrunde, die im Stand der Technik bekannten Systeme weiter zu entwickeln und eine gattungsgemäße Anordnung und ein Verfahren insbesondere im Sinne einer definierten Einstechtiefe zu optimieren, wobei ein Erfindungsziel auch in einer vereinfachten und hygienisch vorteilhaften Systemauslegung besteht.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch einen Hautstraffer als integraler Bestandteil des Stechelements die Haut im Einwirkungsbereich des Stechorgans einzudrücken. Dementsprechend wird erfindungsgemäß vorgeschlagen, dass an dem Träger ein die Haut im Bereich der Einstichstelle straffendes Vorspannmittel angebracht ist, welches ein bei der Stechbewegung auf die Haut aufstoßendes und dabei entgegen dem Stechorgan zurück bewegliches Kontaktglied aufweist. Das Kontaktglied liefert weitere Auflagepunkte auf der Haut, so dass eine bessere Druckverteilung in der Region der Einstichstelle herrscht. Das Kontaktglied sorgt damit für eine Verminderung der Hautverdrängung durch das Stechorgan. Das Kontaktglied bewirkt eine konkave oder konvexe Hautwölbung an der Einstichstelle, so dass durch das auftreffende Stechorgan eine Verminderung der Hautverdrängung erfolgt und sofort der Stichkanal erzeugt wird. Die Freigabe des Stechorgans erfolgt unter relativer Rückbewegung des an der Haut anliegenden Kontaktglieds, wobei sich durch die Hautstraffung direkt an der Einstichstelle der Einfluss hautspezifischer Schwankungen auf die Stechtiefe weitgehend eliminieren lässt. Zudem kann eine Gerätekontamination mit Körperflüssigkeit durch die integrale Anordnung des Kontaktglieds an dem vorzugsweise disposiblen Stechelement zuverlässig vermieden werden. Ein weiterer wichtiger Vorteil liegt darin, dass die Komplexität des eingesetzten Systems bzw. Geräts und damit auch dessen Baugröße verringert werden kann, wenn die Funktion der Hautstraffung nicht geräteseitig, sondern auf Seiten des Stechelements implementiert wird.

Vorteilhafterweise ist das Kontaktglied im Ausgangszustand dem Stechorgan in Stechrichtung vorgelagert und bei der Stechbewegung auf der Haut gegen den Träger rückbeweglich abgestützt. Auf diese Weise wird durch eine einfache Vorschubbewegung das Eindringen des Stechorgans in vorgespannte Haut sichergestellt. Grundsätzlich könnte es auch genügen, wenn sich das Kontaktglied im Wesentlichen auf gleicher Höhe mit dem Stechorgan befindet, so dass zumindest beim Eintritt in die blutgebende Hautzone die Hautstraffung erfolgt ist.

Eine weitere vorteilhafte Ausführung sieht vor, dass das Vorspannmittel ein das Kontaktglied mit dem Träger verbindendes, elastisch und/oder plastisch komprimierbares und/oder durch Reibschluss gehaltenes Kopplungsteil aufweist. Damit wird eine weitere Vorwärtsbewegung des Stechelements ermöglich, während sich das Kontaktglied bereits in Anlage mit der Haut befindet. Hierbei ist es auch herstellungstechnisch vorteilhaft, wenn das Kopplungsteil durch einen an mindestens einer Biegestelle abbiegbaren bzw. knickbaren Faltarm gebildet ist. Ein zusätzlicher Federweg kann dadurch bereitgestellt werden, dass das Kontaktglied über ein Federelement an dem Träger abgestützt ist.

Um bei der Rückbewegung in eine Sammelposition die Kontaktkraft weitgehend aufzuheben, ist es von Vorteil, wenn das Kontaktglied in einer gegenüber dem Stechorgan proximal zurückgestellten Rückstellposition durch einen Sperrmechanismus, insbesondere eine Klinke selbsttätig arretierbar ist.

Für die Blutgewinnung ist es vorteilhaft, wenn das Kontaktglied beim Zurückziehen des Trägers unter Veningerung oder Aufhebung der Anpresskraft an der Haut mitbeweglich ist. Die Körperflüssigkeitsverdrängung durch das Kontaktglied wird dadurch reduziert.

Herstellungstechnisch und in funktionaler Hinsicht ist es von Vorteil, wenn das Vorspannmittel als integraler Bestandteil vorzugsweise einstückig an dem Träger angebracht ist. Besonders bevorzugt ist es, wenn der Träger, das Stechorgan und das Vorspannmittel als Einwegteil einheitlich aus einem Material gebildet sind. Dies ist auch in hygienischer Hinsicht von Vorteil, weil der Hautkontakt nur mit einem sterilisierbaren, einmal verwendeten Artikel stattfindet.

Eine weitere Verbesserung sieht vor, dass das Stechelement aus einem flachen Substrat gebildet, insbesondere geätzt ist, und dass das Vorspannmittel als Substratteil in der Substratebene angeordnet oder aus dieser herausgebogen ist. Kontaktgliedfederungen können auch durch entsprechendes zweidimensionales Ätzen als Bogen- oder Falt-Elemente aus einem ebenen Substrat herstellungstechnisch vorteilhaft erzeugt werden.

Gemäß einer weiteren vorteilhaften Ausgestaltung besitzt das Kontaktglied eine seitlich neben dem Stechorgan gegen die Haut andrückbare punkt- oder linienförmige Randkontur aufweist. Dies sollte so nahe wie möglich an der Einstichstelle erfolgen, ohne jedoch mit dem Stechorgan zu kollidieren. Entsprechend ist es vorteilhaft, wenn das Stechorgan in einem seitlichen Abstand von weniger als 3 mm, vorzugsweise 1 bis 2 mm von dem Kontaktglied in die Haut einstechbar ist.

Zur Stechtiefenregulierung ist es günstig, wenn an dem Träger ein die Einstechtiefe des Stechorgans begrenzender Anschlag einstückig angebracht bzw. angeformt ist, welcher eine bei der Stechbewegung in definiertem proximalem Abstand zu dem Stechorgan anschlagende Hautkontaktfläche aufweist.

Vorteilhafterweise ist der Anschlag zur Stechtiefenregulierung vorzugsweise durch Biegeverformung oder veränderliche Raststellen in seiner an dem Basisteil vorspringenden Länge in Stechrichtung längenveränderlich.

Das Stechelement kann als einfache Lanzette ausgebildet sein, während für ein gleichzeitiges Sammeln von Körperflüssigkeit eine vorzugsweise kapillaraktive Aufnahmestruktur, die in den Bereich des Stechorgans reicht, von Vorteil ist.

Gegenstand der Erfindung ist auch ein Stechsystem zur Gewinnung von Körperflüssigkeit durch bzw. über die Haut mit einem Stechantrieb und einem damit in einer Stechbewegung vorwärts und zurück bewegbaren erfindungsgemäßen Stechelement.

Eine besonders bevorzugte Variante sieht einen Positionsdetektor zur Erfassung der Position der Haut, vorzugsweise der gestrafften Haut im Zuge der Stechbewegung vor. Auf diese Weise ist eine sehr genaue Stechtiefenbestimmung möglich, ohne dass ein "Leerweg" des Stechorgans aufgrund von Hauteindellung zu Fehlern führt. Die Stechbewegung kann eine Tastbewegung mit Rückbewegung vor dem Eindringen umfassen, oder aber eine nur einmal vor und zurück verlaufende Bewegung.

Hierbei ist es vorteilhaft, wenn der Positionsdetektor die Position der durch das Vorspannmittel gestrafften Haut über das Kontaktglied und/oder das Stechorgan als Fühler abtastet, und wenn der Positionsdetektor eine Kapazitäts- oder Leitfähigkeitsänderung oder eine Kraftänderung beim Abtasten der Haut erfasst. Es versteht sich, dass das Stechorgan eine Detektionsfunktion übernehmen kann, indem es zugleich einen Kapazitäts-, Leitfähigkeits- oder Kraftfühler gegenüber der Hautoberfläche bildet.

Gemäß einer weiteren vorteilhaften Ausführung ist das Stechelement an eine vorzugsweise inkremental arbeitende Wegmesseinheit zur Erfassung der Relativposition von Kontaktglied und Stechorgan angeschlossen.

Für einen definierten und damit möglichst schmerzarmen Stechvorgang ist es von Vorteil, wenn der Stechantrieb eine Einrichtung zur Einstellung der Stechtiefe des Stechorgans bezüglich einer erfassten Referenzposition der Haut, vorzugsweise der gestrafften Haut aufweist.

Ein weiterer Erfindungsaspekt liegt darin, ein System zur Hautdetektion für die Probennahme von Körperflüssigkeit bereitzustellen, bei welchem durch einen Positionsdetektor die Position der Haut bezüglich einer Bewegungsachse eines Stechelements erfasst wird, wobei die Haut durch ein Vorspannmittel des Stechelements bei der Positionserfassung lokal gestrafft wird. Hierbei ist es vorteilhaft, wenn das Stechelement nach der Positionserfassung in eine von der Haut zurückgezogene Stellung bewegt wird und sodann mittels eines Stechantriebs die Stechbewegung ausgeführt wird. Für die Hauterkennung kann das Vorspannmittel durch ein Stechorgan oder ein gesondertes Kontaktglied des Stechelements gebildet sein. Vorteilhafterweise wird die Stechbewegung des Stechelements über einen Stechantrieb zur Einstellung einer definierten Stechtiefe nach Maßgabe der erfassten Hautposition gesteuert.

In verfahrensmäßiger Hinsicht wird die eingangs genannte Aufgabe im Sinne einer Hautdetektion für die Probennahme von Körperflüssigkeit, wobei die Position der Haut bezüglich einer Bewegungsachse eines Stechelements erfasst wird, dadurch gelöst, dass die Haut durch ein Vorspannmittel des Stechelements bei der Positionserfassung lokal gestrafft wird.

Um die Stechbewegung mit hoher Dynamik ausführen zu können; ist es vorteilhaft, wenn das Stechelement nach der Positionserfassung und vor einer Stechbewegung in eine von der Haut zurückgezogene Startstellung bewegt wird. Das Vorspannmittel kann durch ein Stechorgan oder ein davon gesondertes Kontaktglied des Stechelements gebildet sein. In diesem Zusammenhang ist es auch günstig, wenn die Stechbewegung des Stechelements zur Einstellung einer definierten Stechtiefe nach Maßgabe der erfassten Hautposition gesteuert wird.

Im Folgenden wird die Erfindung anhand der in der Zeichnung schematisch dargestellten Ausführungsbeispiele näher erläutert. Es zeigen
- Fig. 1: ein Blutzuckermessgerät mit einem zur Hautstraffung an der Stechstelle ausgebildeten Stechelement in einer schematisch ver- einfachten Darstellung;
- Fig.2 bis 4: weitere Ausführungsformen eines Stechelements mit Hautstraffer;
- Fig. 5: das Stechelement nach Fig. 1 in verschiedenen Positionen bei der Stechbewegung;
- Fig. 6: eine Ausführungsform eines Stechelements mit einrastbarem Hautstraffer in einer Seitenansicht;
- Fig. 7: eine weitere Ausführungsform eines Einrastmechanismus in aus- schnittsweiser Darstellung;
- Fig. 8: einen Hautsensor an einem Stechelement in ausschnittsweiser Ansicht im Ausgangszustand und Stechzustand;
- Fig. 9: einen zusätzlichen Ring bzw. Fingerhalter in Verbindung mit dem Stechelement;
- Fig. 10 und 11: eine Darstellung von zwei verschiedenen Stechmetho- den jeweils in der Ausgangs- und Endstellung;
- Fig. 12 und 13: Stechelemente mit verstellbarem Abstandshalter zur Stechtiefenregulierung in schaubildlicher Darstellung.

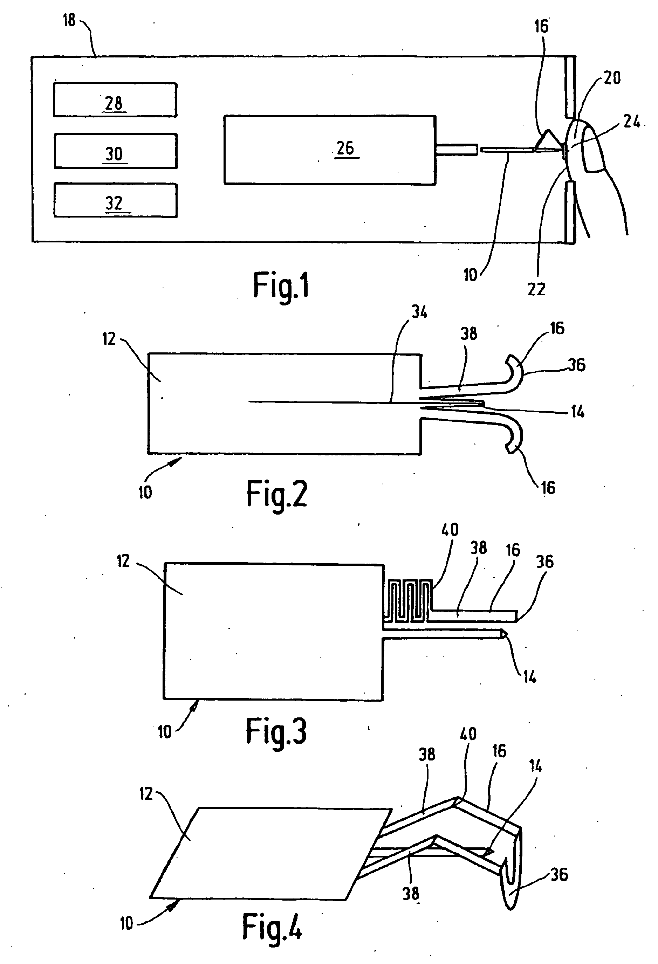

Die in der Zeichnung dargestellten Stechelemente 10 besitzen einen Träger bzw. ein Basisteil 12 als Halter, ein an dem Halter in Stechrichtung (distal) abstehendes, als Spitze ausgebildetes Stechorgan 14 und ein neben dem Stechorgan auf die Haut aufsetzbares Vorspannmittel 16 als Hautstraffer. Gemäß Fig. 1 lassen sich solche Stechelemente 10 als Einmalartikel (so genanntes "Disposable") in ein Handgerät 18 einsetzen, um in einer Stechbewegung Körperflüssigkeit, d.h. Blut, gegebenenfalls auch Gewebeflüssigkeit speziell für Blutzucker-Selbsttests zu gewinnen. Hierbei wird von dem Benutzer ein Körperteil, insbesondere die Fingerbeere 20 auf eine Geräteöffnung aufgesetzt. Sodann wird in einer vorwärts und zurück verlaufenden Stechbewegung gegen die in der Geräteöffnung liegende Hautpartie 22 mittels des Stechorgans 14 an einer Einstichstelle eine geringe Blutmenge gewonnen und vorzugsweise im Gerät 18 analysiert.

Zu diesem Zweck weist das Gerät 18 einen das eingesetzte Stechelement 10 bewegenden Stechantrieb 26, einen Hautdetektor 28, eine damit zusammenwirkende Einrichtung zur Stechtiefeneinstellung 30 und weitere Gerätebaugruppen wie eine Analyseeinheit 32 auf. Das auf dem Testelement 10 gesammelte Blut kann damit für eine Blutzuckerbestimmung vor Ort in einem automatischen Messablauf verwertet werden. Anschließend wird das gebrauchte Testelement entsorgt und vorzugsweise aus einem Gerätemagazin ein neues Testelement bereitgestellt, so dass eine möglichst hygienische Handhabung gewährleistet ist.

Das in Fig. 2 gezeigte Testelement 10 ist einheitlich aus einem flachen Substrat, beispielsweise Edelstahlblech geätzt, wobei das Stechorgan 14 und das Vorspannmittel 16 als in der Ebene des Substrats liegende integrale Bestandteile zusammen mit dem Basisteil 12 freigeätzt sind. Auf diese Weise können einstückige, aus einem Teil gebildete Stechelemente in einem für die Massenfertigung geeigneten einheitlichen Verfahrensablauf hergestellt werden. Um zusätzlich eine Sammelfunktion für die an der Einstichstelle gewonnene Körperflüssigkeit zu integrieren, ist ein längsseitig halboffener Kapillarkanal 34 über das Basisteil 12 bis in den Bereich des Stechorgans 14 geführt.

Das Vorspannmittel 16 ist durch ein bei der Stechbewegung seitlich vor dem Stechorgan 14 auf die Haut auftreffendes Kontaktglied 36 und ein das Kontaktglied mit dem Träger 12 verbindendes Kopplungsteil 38 gebildet. Auf diese Weise wird das Vorspannmittel 16 als integrale Struktur mit dem Träger 12 mitbewegt. Das Kontaktglied 36 wird bei der Vorwärtsbewegung des Stechelements 10 vor dessen Hautkontakt mit einer punkt- oder linienförmigen Randkontur auf die Haut 22 aufgesetzt. Beim weiteren Vorschub wird das Kopplungsteil 38 durch das in Anlage auf der Haut relativ zu dem Stechorgan 14 zurückbewegte Kontaktglied 36 elastisch und/oder plastisch in seiner distalen Länge verformt, so dass das Stechelement 14 in die zuvor gestraffte Haut einsticht, wie es nachstehend näher erläutert wird.

Bei dem in Fig. 3 gezeigten Ausführungsbeispiel wird das Kontaktglied 36 ebenfalls in geringem seitlichem Abstand und in Stechrichtung vor dem Stechorgan 14 gehalten, wobei ein Federelement 40 eine begrenzte Rückbewegung gegen das Basisteil 12 beim Stechvorschub erlaubt. Um eine Hautstraffung nahe der Einstichstelle zu erreichen, beträgt der seitliche Abstand zwischen dem Kontaktglied 36 und dem Stechorgan 14 in der Wirkstellung weniger als 3 mm, vorzugsweise 1 bis 2 mm.

Während bei den Ausführungen nach Fig. 2 und 3 das Vorspannmittel 16 in der Substratebene angeordnet ist, sieht das Ausführungsbeispiel nach Fig. 4 einen Doppel-Faltarm 38 als Kopplungsteil vor, welcher an einer aus der Substratebene heraus abknickbaren Biegestelle 40 eine distale Verkürzung gegenüber dem Stechorgan 14 erlaubt.

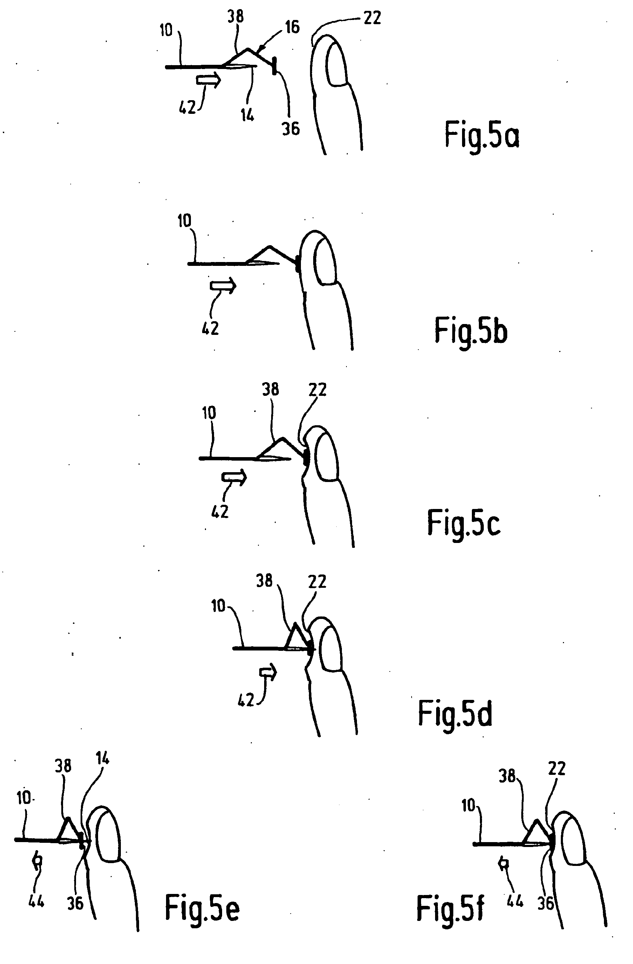

Fig. 5 veranschaulicht die Hautstraffung beim Stech- und Sammelvorgang in verschiedenen Bewegungsstadien. Bei der Vorwärtsbewegung (Pfeil 42) befindet sich das Kontaktglied 36 des Vorspannmittels bzw. Hautstraffers 16 vor dem Stechorgan (Fig. 5a) und trifft somit nahe der vorgesehenen Einstichstelle auf die Haut 22 auf (Fig. 5b). Beim weiteren Vorschub wird die Haut 22 mit definierter Kraft verdrängt, wobei der Faltarm 38 komprimiert wird (Fig. 5c), bis schließlich der Nadeleinstich in die vorgespannte Haut 22 erfolgt (Fig. 5d).

Nach Erreichen der vorgesehenen und gegenüber der gestrafften Haut definierten Einstechtiefe wird das Stechelement in einer Rückbewegung (Pfeil 44) auf eine weniger tief eingestochene Sammelposition zurückgezogen. Sofern der Faltarm 38 zuvor plastisch verformt oder durch einen Sperrmechanismus zurückgehalten wurde, folgt das Kontaktglied 36 der Rückbewegung, wie es in Fig. 5e gezeigt ist. Dabei wird die Andrückkraft weitgehend aufgehoben, und die Haut 22 im Bereich der Einstichstelle relaxiert. Dadurch wird weniger Körperflüssigkeit in der betreffenden Hautpartie verdrängt, und es kann in kurzer Zeit eine ausreichende Flüssigkeitsmenge (Mikroliter oder weniger) gewonnen werden. Bei der in Fig. 5f gezeigten Alternative wurde der Faltarm 38 beim Einstechen nicht plastisch verformt, sondern nur elastisch um eine bestimmte Länge zusammengedrückt. Beim Zurückziehen verbleibt somit das Kontaktglied 36 auf der Haut 22, während die Rückstellkraft des Faltarms 38 sich verringert. Damit ist auch hier die Verdrängung der Körperflüssigkeit im Körperteil kleiner als bei der maximalen Einstechtiefe.

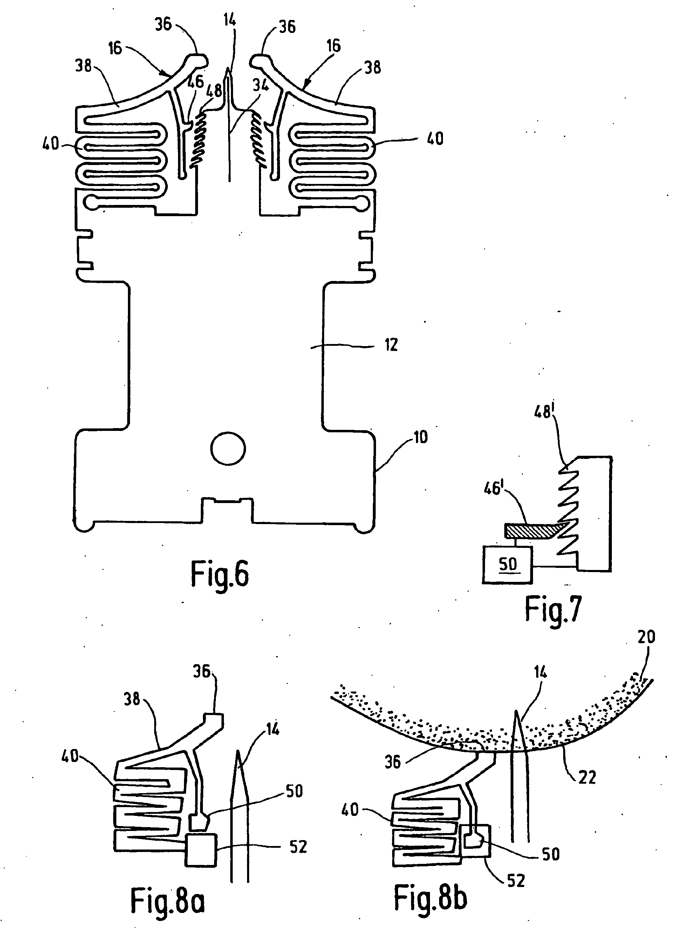

Bei den in den folgenden Figuren gezeigten Ausführungsformen sind vorstehend bereits beschriebene Teile mit den gleichen Bezugszeichen versehen. Gemäß Fig. 6 ist zusätzlich an dem Kopplungsteil 38 eine Sperrklinke 46 angeformt, welche in eine Verzahnung 48 an dem Basisteil 12 zur Sicherung der erreichten Rückstellposition des Kontaktglieds 36 einhakbar ist. Dadurch wird bei maximalem Vorschub des Stechelements 10 und entsprechender elastischer Rückstellung des Vorspannmittels 16 in Anlage mit der Haut 22 eine Arretierung erreicht, so dass ähnlich der Ausführung nach Fig. 5e der komprimierte Vorspanner 16 bei der Rückbewegung in die Sammelposition nicht mehr gegen die Haut 22 andrückt.

Entsprechend Fig. 7 ist es auch denkbar, dass eine geräteseitige Rastklinke 46' in eine Verzahnung 48' des Vorspannmittels 16 richtungsabhängig arretierend eingreift. Zweckmäßig besitzt diese Klinke 46' eine größere Dicke als die Verzahnung 48', so dass gewisse Höhentoleranzen der Relativpositionierung ausgeglichen werden können. Denkbar sind hierzu auch geeignete Ausbiegungen der Verzahnung 48' aus der Substratebene. Grundsätzlich ist es mit einer solchen Anordnung auch möglich, über eine Wegmesseinheit 50 die Anzahl der Einrastklicks (beispielsweise mittels eines elektrischen Impuls-Zählers) zu erfassen und auf diese Weise die Relativverschiebung zwischen Kontaktglied 36 und Stechorgan 14 zu ermitteln und im Sinne einer Stechtiefenbestimmung auszuwerten.

Fig. 8 veranschaulicht eine Ausführungsform, bei der mittels des Hautdetektors 28 über das Kontaktglied 36 die Position der gestrafften Haut in Stechrichtung kapazitiv erfasst wird. Hierfür ist an dem Kopplungsteil 38 ein Elektrodenarm 50 angeordnet, welcher mit einer gerätefesten Gegenelektrode 52 als wegsensitive Kondensatoranordnung zusammenwirkt. Wie aus Fig. 8 zu ersehen, wird in der Vorschubstellung bei maximaler Hautstraffung die maximale Kapazität erreicht. Daraus lässt sich auch die Relatiwerschiebung zu dem Stechorgan 14 und damit dessen tatsächliche Einstechtiefe bestimmen, beispielsweise indem durch eine Kalibrierung empirische Vergleichswerte gewonnen werden. Die Stechtiefenerfassung kann noch während der Vorwärtsbewegung als Eingangssignal für die Einrichtung zur Stechtiefeneinstellung 30 benutzt werden, welche den Stechantrieb 26 entsprechend steuert bzw. regelt.

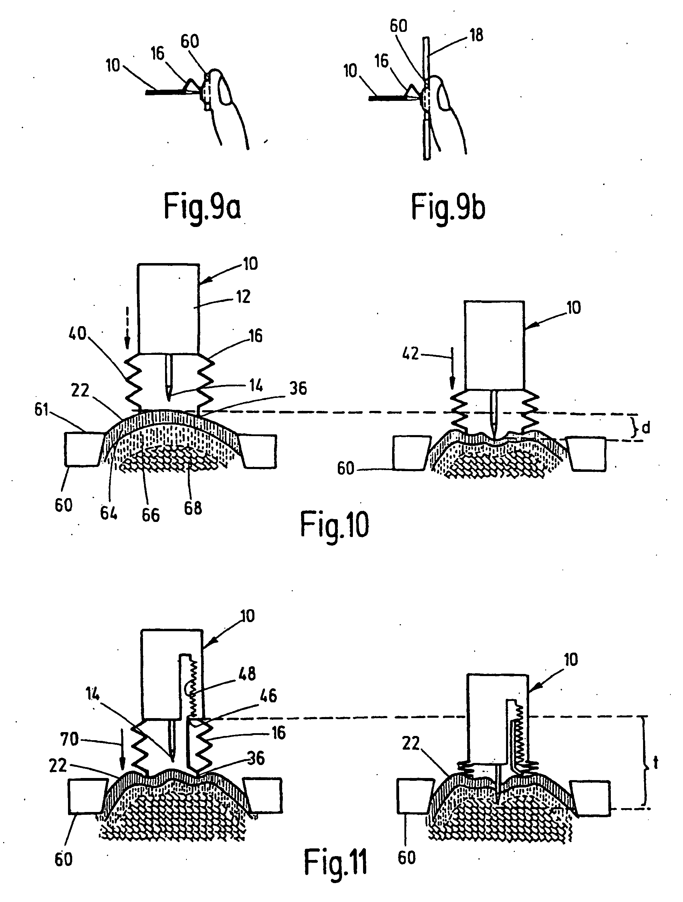

Fig. 9a zeigt das Stechelement 10 in Kombination mit einem Konus oder Anpressring 60 als Fingeraufnahme. Eine solche Fingeraufnahme 60 kann gemäß Fig. 9b auch in eine Öffnung eines in Fig. 1 dargestellten Geräts 18 eingesetzt werden. In jedem Fall wirkt das Vorspannmittel 16 innerhalb der durch die Aufnahme 60 gebildeten Ringbegrenzung, wodurch bereits eine Vorfixierung der Haut 22 erreicht wird.

Wie in Fig. 10 näher veranschaulicht, bewirkt das Auftreffen des Vorspannmittels 16 auf die Haut 22 eine bessere Druckverteilung beim Stechvorgang. Dies kommt dadurch zustande, dass mehrere Auflagepunkte oder -linien ggf. innerhalb eines zusätzlichen Fingerrings 60 vorhanden sind. Dadurch ist die maximale Verdrängung d der Haut 22 vor dem Eindringen des Stechorgans 14 deutlich geringer als ohne Vorspannmittel. Dies gilt für jeden Hauttyp, ob weich, mittel oder hart. Allgemein ist hier zu berücksichtigen, dass die Haut schichtförmig aufgebaut ist, mit dem so genannten Stratum Comeum 64 als oberster Schicht, der anschließenden Epidermis 66 und der darunter liegenden blutgebenden Zone 68.

Zur Gewinnung von Körperflüssigkeit kann zunächst eine Hautberührung des Kontaktglieds 36 erfasst werden, wie es in der linken Hälfte von Fig. 10 dargestellt ist. Das Kontaktglied 36 stellt hierbei eine Referenz für die Hautoberfläche dar, nicht jedoch notwendigerweise einen messenden Sensor. Vielmehr kann die sensorische Erfassung gemäß Fig. 8 erfolgen, oder aber über die antriebsseitige Erfassung einer Veränderung der erforderlichen Vortriebskraft. In jedem Fall ist es wichtig, dass das Stechelement 10 genügend genau gefertigt ist, um die Relativdistanz zwischen Kontaktglied 36 und Stechorgan 14 einbeziehen zu können. Ausgehend von dieser Ausgangsposition erfolgt dann der Stechhub vorzugsweise mit einer vom Benutzer voreingestellten Distanz bzw. Tiefe, wobei aufgrund der vorteilhaften Wirkung des Vorspannmittels 16 nur eine geringe, weitgehend invariante Hautverdrängung d auftritt.

Denkbar ist es auch, dass eine geräteseitige Referenzposition, beispielsweise die Anlagefläche 61 des Rings als Ausgangsposition für den Stechhub gewählt wird, oder aber dass gänzlich auf die Positionserkennung verzichtet werden kann, wenn die maximale Verdrängung d der Haut vor dem Nadeleindringen mit Vorspannmittel 16 deutlich geringer ist als die Schwankungen der Hautwölbung am Ring 60.

Fig. 11 veranschaulicht eine weitere Stechmethode mit einer Krafterkennung am Vorspannmittel 16. Hierbei wird in einer Tastbewegung 70 der Verfahrweg bis zum Auftreten einer vorgegebenen Rückstellkraft bestimmt, beispielsweise anhand eines ersten Rastklicks an der Verzahnung 48, oder bei Erreichen eines kapazitiven Messfeldes 52. Dieser unterschiedliche Verfahrweg bei definierter Vorspannkraft kann als Information über den Hauttyp (weich, mittel, hart) genutzt werden, um für das Einstechen und Eindringen die erforderliche Tiefe t zu bestimmen. Das Kontaktglied 16 als Taster ist also auch hierbei integraler Bestandteil des Disposables 10.

Die Krafterfassung kann vor dem Stechen in einem separaten Abtastvorgang erfolgen oder aber als Teil des Stechvorgangs mit entsprechend schneller Auswertung des Kraftsignals Antriebssteuerung in Echtzeit.

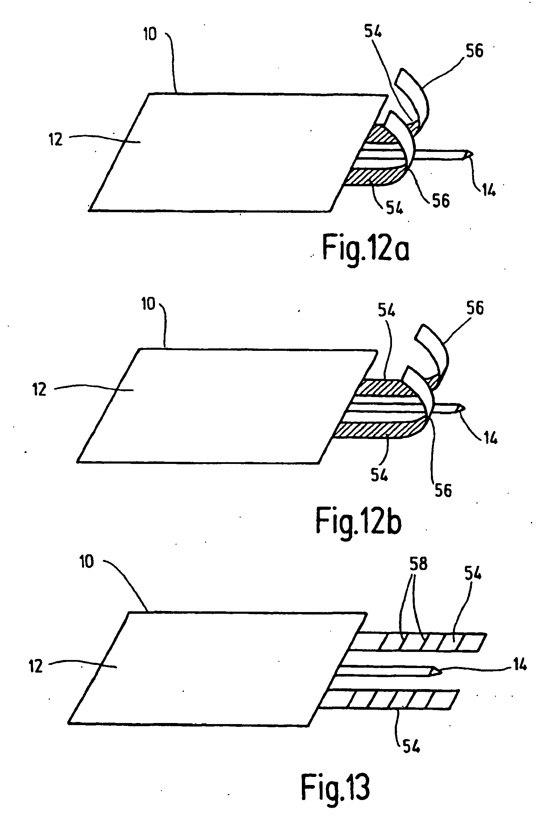

In Fig. 12 und 13 sind Ausführungsformen eines Stechelements 10 mit längenverstellbaren Anschlägen gezeigt, welche eine vorgegebene Stechtiefe des Stechorgans 14 in dem Körperteil definieren. Diese Anschläge 54 sind als integrierte Strukturen an dem Stechelement 10 angeformt und mit einer proximal hinter dem Stechorgan 14 liegenden Anschlagfläche 56 versehen. Wie aus Fig. 12a und 12b ersichtlich, ist die Anschlagposition gegenüber dem Stechelement 14 durch eine Biegeverformung, die im Gerät 18 vor dem Stechen ausgeführt wird, variabel, so dass entsprechend eine an das Körperteil angepasste Stechtiefe fest an dem Stechelement voreinstellbar ist. Damit kann beispielsweise unterschiedlichen Haut-Typen Rechnung getragen werden, um zuverlässig und zugleich schmerzarm die Blut gebende Zone zu erreichen.

In der Ausführung nach Fig. 13 sind an den Anschlägen 54 eine Mehrzahl von Knickstellen 58 vorgeformt, so dass die Biegeanpassung der Anschlagposition vereinfacht wird. Alternativ kann auch eine Längenanpassung durch Abbrechen von Segmenten erreicht werden. Die Einstellung wird im Gerät 18 an einem einzigen Teil, d.h. dem Stechelement 10 vorgenommen, so dass nicht mehrere Baueinheiten eingestellt werden müssen. Denkbar sind auch Varianten mit einer einrastbaren Verzahnung ähnlich der zu Fig. 6 beschriebenen Rückstellsicherung.

## Patentansprüche

1. Stechsystem zur Gewinnung von Körperflüssigkeit durch die Haut (22), insbesondere als Einwegteil für Blutzuckertests, mit einem Träger (12) und einem starr daran abstehenden, in einer Stechbewegung in die Haut (22) einstechbaren Stechorgan (14) sowie einer als - Konus oder Anpressring die Haut abstützenden Fingeraufnahme (60), wobei an dem Träger (12) ein die Haut (22) im Bereich der Einstichstelle straffendes Vorspannmittel (16) angebracht ist, welches ein bei der Stechbewegung in die Fingeraufnahme eingreifendes, auf die Haut (22) aufstoßendes und dabei entgegen dem Stechorgan (14) zurück bewegliches Kontaktglied (36) aufweist, und mit einer Aufnahmestruktur (34), welche bei einer Rückbewegung des Stechelements in einer Sammelposition die Körperflüssigkeit aufnimmt, wobei das Kontaktglied (36) so mitbewegbar ist, dass die Anpresskraft an der Haut in der Sammelposition verringert oder aufgehoben ist.

2. Stechsystem nach Anspruch 1, **dadurch gekennzeichnet, dass** das Kontaktglied (36) im Ausgangszustand dem Stechorgan (14) in Stechrichtung seitlich vorgelagert, auf gleicher Höhe befindlich oder nachgelagert ist und bei der Stechbewegung in Kontakt mit der Haut gegen den Träger (12) rückstellbar ist.

3. Stechsystem nach Anspruch 1 oder 2, **dadurch gekennzeichnet, dass** das Vorspannmittel (16) ein das Kontaktglied (36) mit dem Träger (12) verbindendes, elastisch und/oder plastisch komprimierbares und/oder durch Reibschluss gehaltenes Kopplungsteil (38) aufweist.

4. Stechsystem nach Anspruch 3, **dadurch gekennzeichnet, dass** das Kopplungsteil (38) durch einen an mindestens einer Biegestelle (40) abbiegbaren Faltarm gebildet ist.

5. Stechsystem nach einem der Ansprüche 1 bis 4, **dadurch gekennzeichnet, dass** das Kontaktglied (36) über ein Federelement (40) an dem Träger (12) abgestützt ist.

6. Stechsystem nach einem der Ansprüche 1 bis 5, **dadurch gekennzeichnet, dass** das Kontaktglied (36) in einer gegenüber dem Stechorgan (14) proximal zurückgestellten Rückstellposition durch einen Sperrmechanismus (46,48), insbesondere eine Klinke arretierbar ist.

7. Stechsystem nach einem der Ansprüche 1 bis 6, **dadurch gekennzeichnet, dass** das Vorspannmittel (16) als integraler Bestandteil vorzugsweise einstückig an dem Träger (12) angebracht ist.

8. Stechsystem nach einem der Ansprüche 1 bis 7, **dadurch gekennzeichnet, dass** der Träger (12), das Stechorgan (14) und das Vorspannmittel (16) als Einwegteil (10) einheitlich aus einem Material gebildet sind.

9. Stechsystem nach einem der Ansprüche 1 bis 8, **dadurch gekennzeichnet, dass** es aus einem flachen Substrat gebildet, insbesondere geätzt ist, und dass das Vorspannmittel (16) als Substratteil in der Substratebene angeordnet oder aus dieser herausgebogen ist.

10. Stechsystem nach einem der Ansprüche 1 bis 9, **dadurch gekennzeichnet, dass** das Kontaktglied (36) eine seitlich nahe dem Stechorgan (14) gegen die Haut (22) andrückbare punkt- oder linienförmige Randkontur aufweist.

11. Stechsystem nach einem der Ansprüche 1 bis 10, **dadurch gekennzeichnet, dass** das Stechorgan (14) in einem seitlichen Abstand von weniger als 3 mm, vorzugsweise 1 bis 2 mm von dem Kontaktglied (36) in die Haut (22) einstechbar ist.

12. Stechsystem nach einem der Ansprüche 1 bis 11, **dadurch gekennzeichnet, dass** die Fingeraufnahme (60) beim Abstützen der Haut eine Hautwölbung begrenzt, und dass das Vorspannmittel (16) bei der Stechbewegung mit seinem Kontaktglied (36) auf die Hautwölbung auftrifft und diese reduziert.

13. Stechsystem nach einem der Ansprüche 1 bis 12, **dadurch gekennzeichnet**, die Fingeraufnahme in eine Öffnung eines Geräts, insbesondere eines Handgeräts (18) eingesetzt ist.

## Claims

1. Lancing system for collecting body fluid through the skin (22) in particular as a disposable part for blood sugar tests comprising a support (12) and a lancing member (14) which rigidly projects therefrom and can pierce the skin (22) in a lancing movement and a finger receiver (60) supporting the skin and formed as a cone or pressure ring, wherein a pretensioning means (16) which tightens the skin (22) in the area of the puncture site and which has a contact member (36) that engages into the finger receiver and impacts the skin (22) during the lancing movement and can move back during this process in the opposite direction to the lancing member (14), is attached to the support (12), and further comprising a receiving structure (34) which receives the body fluid in a collecting position during a backward movement of the lancing element, wherein the contact member (36) can also be moved in such a manner that the contact pressure on the skin in the collecting position is reduced or removed.

2. Lancing system according to claim 1, **characterized in that** in the initial state the contact member (36) is positioned in the direction of lancing to the side and in front of the lancing member (14), or it is located at the same level or downstream thereof and can be reset against the support (12) during the lancing movement while in contact with the skin.

3. Lancing system according to claim 1 or 2, **characterized in that** the pretensioning means (16) has an elastically and/or plastically compressible coupling member (38) and/or one that is held by frictional lock which connects the contact member (36) with the support (12).

4. Lancing system according to claim 3, **characterized in that** the coupling member (38) is formed by a folding arm which can be bent at at least one bending point (40).

5. Lancing system according to one of the claims 1 to 4, **characterized in that** the contact member (36) is supported on the support (12) via a spring element (40).

6. Lancing system according to one of the claims 1 to 5, **characterized in that** the contact member (36) can be locked in a reset position that is proximally set back relative to the lancing member (14) by means of a locking mechanism (46, 48) and in particular a catch.

7. Lancing system according to one of the claims 1 to 6, **characterized in that** the pretensioning means (16) is attached as an integral component and preferably as one piece to the support (12).

8. Lancing system according to one of the claims 1 to 7, **characterized in that** the support (12), the lancing member (14) and the pretensioning means (16) are formed uniformly from one material as a disposable part (10).

9. Lancing system according to one of the claims 1 to 8, **characterized in that** it is formed from a flat substrate and is in particular etched and that the pretensioning means (16) is arranged as a part of the substrate in the substrate plane or is bent out of this plane.

10. Lancing system according to one of the claims 1 to 9, **characterized in that** the contact member (36) has a point shaped or linear edge contour that can be pressed against the skin (22) laterally next to the lancing member (14).

11. Lancing system according to one of the claims 1 to 10, **characterized in that** the lancing member (14) can be inserted into the skin (22) at a lateral distance of less than 3 mm and preferably of 1 to 2 mm from the contact member (36).

12. Lancing system according to one of the claims 1 to 11, **characterized in that** the finger receiver (60) defines a skin bulge when supporting the skin, and that during the lancing movement the pretensioning means (16) by means of its contact member (36) impacts on the skin bulge and reduces it.

13. Lancing system according to one of the claims 1 to 12, **characterized in that** the finger receiver is inserted into an opening of a device, in particular of a hand held device (18).

## Revendications

1. Système de piquage destiné à collecter du liquide corporel à travers la peau (22), en particulier en tant que partie à usage unique pour tests de glycémie, comprenant un support (12) et un élément de piqûre (14) en saillie rigide au niveau de celui-ci et qui peut percer la peau (22) au cours d'un mouvement de piqûre, ainsi qu'un récepteur de doigt (60) sous forme de cône ou d'anneau de pression servant d'appui pour la peau, un moyen de précontrainte (16) étant monté sur le support (12), qui sert à tendre la peau (22) au niveau de l'endroit de la piqûre et qui présente un élément de contact (36) qui s'introduit dans le récepteur de doigt lors du mouvement de piqûre, percute la peau (22) et est alors apte à se déplacer vers l'arrière en sens inverse de l'élément de piqûre (14), et comprenant en outre une structure de recueil (34) qui lors d'un déplacement vers l'arrière de l'élément de piqûre collecte le liquide corporel dans une position de collecte, ledit élément de contact (36) étant apte à se déplacer en même temps, de telle sorte que la pression de contact exercée sur la peau dans la position de collecte est diminuée ou annulée.

2. Système de piquage selon la revendication 1, **caractérisé en ce qu'**à l'état initial, l'élément de contact (36) est placé, dans le sens de la piqûre, latéralement en amont, au niveau ou en aval de l'élément de piqûre (14) et lors du mouvement de piqûre, peut se repositionner contre le support (12) tout en restant en contact avec la peau.

3. Système de piquage selon la revendication 1 ou 2, **caractérisé en ce que** le moyen de précontrainte (16) présente une pièce de couplage (38) qui relie l'élément de contact (36) au support (12), est compressible élastiquement et/ou plastiquement et/ou est maintenue par friction.

4. Système de piquage selon la revendication 3, **caractérisé en ce que** la pièce de couplage (38) est formée par un bras pliant pouvant se plier à au moins un point de pliage (40).

5. Système de piquage selon l'une des revendications 1 à 4, **caractérisé en ce que** l'élément de contact (36) s'appuie sur le support (12) par l'intermédiaire d'un élément ressort (40).

6. Système de piquage selon l'une des revendications 1 à 5, **caractérisé en ce que** l'élément de contact (36) peut être immobilisé dans une position de rappel en retrait proximal par rapport à l'élément de piqûre (14), grâce à un mécanisme de blocage (46, 48), en particulier un cliquet.

7. Système de piquage selon l'une des revendications 1 à 6, **caractérisé en ce que** le moyen de précontrainte (16), en tant que partie intégrante, est monté de préférence d'un seul tenant sur le support (12).

8. Système de piquage selon l'une des revendications 1 à 7, **caractérisé en ce que** le support (12), l'élément de piqûre (14) et le moyen de précontrainte (16), en tant que partie à usage unique (10), sont constitués de manière homogène d'un même matériau.

9. Système de piquage selon l'une des revendications 1 à 8, **caractérisé en ce qu'**il est réalisé, notamment par gravure, à partir d'un substrat plat, et **en ce que** le moyen de précontrainte (16), en tant que partie du substrat, est disposé dans le plan du substrat ou est plié hors de celui-ci.

10. Système de piquage selon l'une des revendications 1 à 9, **caractérisé en ce que** l'élément de contact (36) présente un contour périphérique en forme de point ou de ligne, apte à s'appuyer latéralement contre la peau (22), près de l'élément de piqûre (14).

11. Système de piquage selon l'une des revendications 1 à 10, **caractérisé en ce que** l'élément de piqûre (14) peut s'enfoncer dans la peau (22) à une distance latérale de moins de 3 mm, de préférence 1 à 2 mm, de l'élément de contact (36).

12. Système de piquage selon l'une des revendications 1 à 11, **caractérisé en ce que** le récepteur de doigt (60), lorsqu'il soutient la peau, limite un bombement de la peau, et **en ce que** le moyen de précontrainte (16), lors du mouvement de piqûre, percute le bombement de la peau avec l'élément de contact (36) et réduit celui-ci.

13. Système de piquage selon l'une des revendications 1 à 12, **caractérisé en ce que** ledit récepteur de doigt est placé dans une ouverture d'un appareil, en particulier d'un appareil portatif (18).
